## Europäisches Patentamt

## European Patent Office

## Office européen des brevets

(19)

(11) Veröffentlichungsnummer: **0 346 470**
**A1**

(12) **EUROPÄISCHE PATENTANMELDUNG**

Veröffentlicht nach Art. 158 Abs. 3 EPÜ

(21) Anmeldenummer: **88901161.5**

(22) Anmeldetag: **14.12.87**

Daten der zugrundeliegenden internationalen Anmeldung:

(86) Internationale Anmeldenummer:
**PCT/SU87/00144**

(87) Internationale Veröffentlichungsnummer:
**WO89/05789 (29.06.89 89/14)**

(51) Int. Cl.³: **C 07 C 69/732**
**C 07 C 67/52**

(43) Veröffentlichungstag der Anmeldung:
**20.12.89 Patentblatt 89/51**

(84) Benannte Vertragsstaaten:
**BE DE FR GB IT NL**

(71) Anmelder: **NAUCHNO-ISSLEDOVATELSKY INSTITUT KHIMIKATOV DLYA POLIMERNYKH MATERIALOV**
**ul. Montazhnikov, 3,**
**Tambov, 392680(SU)**

(72) Erfinder: **GLUSHKOVA, Ljudmila Vasilievna**
**ul. Michurinskaya, 143-80**
**Tambov, 392032(SU)**

(72) Erfinder: **BELOVA, Svetlana Jurievna**
**2-i Pochtovy proezd, 3-51**
**Tambov, 392028(SU)**

(72) Erfinder: **PARAMONOV, Vladimir Ivanovich**
**ul. Michurinskaya, 143-8**
**Tambov, 392032(SU)**

(72) Erfinder: **GORBUNOV, Boris Nikolaevich**
**ul. Stepana Razina, 11/13-9**
**Tambov, 392000(SU)**

(72) Erfinder: **EGIDIS, Felix Mikhailovich**
**ul. Kolkhoznaya, 91a-20**
**Tambov, 392018(SU)**

(74) Vertreter: **von Füner, Alexander, Dr. et al,**
**Patentanwälte v. Füner, Ebbinghaus, Finck Mariahilfplatz 2 & 3**
**D-8000 München 90(DE)**

(54) **EXTRAKTIONSVERFAHREN VON PENTAERYTHRYL-TETRAKIS-3-(3,5-DITERT. BUTYL-4-OXYPHENYL)PROPIONATEN AUS EINER REAKTIONSMASSE WÄHREND DEREN HERSTELLUNG.**

(57) Das Verfahren zur Ausscheidung von Pentaerythryltetrakis- [3-(3,5-ditert.butyl-4-oxyphenyl)propionat] aus einer Reaktionsmasse, die bei der Synthese des genannten Produktes durch Umesterung des Methylesters der 3-(3,5-ditert.Butyl-4-oxyphenyl)propionsäure mit Pentaerythrit in Gegenwart eines alkalischen Katalysators gewonnen wird und die sich aus dem Endprodukt, den nichtumgesetzten Ausgangsreaktionsteilnehmern, den Nebenprodukten der Reaktion, den harzartigen Oxydationsprodukten und den Rückständen des alkalischen Katalysators zusammensetzt, besteht darin, daß man die Reaktionsmasse zum Zyklohexan (Volumenverhältnis der Reaktionsmasse zum Zyklohexan gleich 1:1,2 bis 1:2) bei einer Temperatur der Reaktionsmasse von 100 bis 120°C behandelt, den nichtumgesetzten Ausgangspentaerythrit und Rückstände des alkalischen Katalysators bei einer Temperatur von 60 bis 75°C durch Filterung abscheidet, wodurch man eine Zyklohexanlösung aus dem Endprodukt, dem nichtumgesetzten Ausgangsmethylester, den Nebenprodukten der Reaktion und den harzartigen Oxydationsprodukten erhält. Die Zyklohexanlösung kühlt man auf eine Temperatur von 15 bis 20°C ab, indem man kristallinen Rückstand einer Einschlußverbindung des Endproduktes mit Zyklohexan erhält. Der Rückstand wird durch Filterung abgeschieden und in normalen Kohlenwasserstoffen bei einer Temperatur von 45 bis 75°C und bei einem Massenverhältnis des Rückstandes zu den Kohlenwasserstoffen von 1:1 bis 1:2 aufgelöst, dabei zersetzt sich die Einschlußverbindung unter Anfallen des Endproduktes. Man scheidet das Endprodukt durch Kristallisation aus den genannten Kohlenwasserstoffen bei einer Temperatur von 15 bis 20°C ab. Das Endprodukt stellt ein hocheffektives nichttoxisches Antioxydationsmittel für Polymerstoffe dar.

EP 0 346 470 A1

VERFAHREN ZUR AUSSCHEIDUNG VON PENTAERYTHRYL-TETRAKIS-
[3-(3,5-DITERT.-BUTYL-4-OXYPHENYL) PROPIONAT] AUS EINER
REAKTIONSMASSE, DIE BEI DER SYNTHESE DES GENANNTEN PRO-
DUKTES GEWONNEN WIRD

### Gebiet der Technik

Die Erfindung bezieht sich auf das Gebiet der organischen Synthese und betrifft insbesondere Verfahren zur
Ausscheidung von Pentaerythryl-tetrakis- [3-(3,5-ditert.
butyl-4-oxyphenyl)propionat] aus einer Reaktionsmasse,
die bei der Synthese des genannten Produktes durch Umesterung des Methylesters der 3-(3,5-ditert.butyl-4-oxyphenyl)
propionsäure mit Pentaerythrit in Gegenwart eines alkalischen Katalysators gewonnen wird und die sich aus dem Endprodukt, aus den nichtumgesetzten Ausgangsreaktionsteilnehmern, Nebenprodukten der Reaktion, aus harzartigen Oxydationsprodukten und aus Rückständen des alkalischen Katalysators zusammensetzt.

### Stand der Technik

Bekannt ist ein Verfahren zur Ausscheidung von Pentaerythryl-tetrakis- [3-(3,5-ditert.butyl-4-oxyphenyl)propionat] aus einer Reaktionsmasse, die bei der Synthese des
genannten Produktes durch Umesterung des Methylesters der
3-(3,5-ditert.Butyl-4-oxyphenyl)propionsäure mit Pentaerythrit in Gegenwart eines alkalischen Katalysators, Lithiumhydrids, gewonnen wird und die sich aus dem Endprodukt, den nichtumgesetzten Ausgangsreaktionsteilnehmern,
den Nebenprodukten der Reaktion, den harzartigen Oxydationsprodukten und den Rückständen des alkalischen Katalysators
zusammensetzt, besteht darin, daß man der genannten Reaktionsmasse Benzol zusetzt, das angefallene Gemisch mit
wasserfreier Essigsäure behandelt und die feste Phase durch
Filtern abscheidet, die ein Gemisch aus Lithiumazetat mit
dem nichtumgesetzten Ausgangspentaerythrit darstellt. Die
Benzollösung, die das Endprodukt, den nichtumgesetzten
Ausgangsmethylester der 3-(3,5-ditert.Butyl-4-oxyphenyl)
propionsäure, Nebenprodukte der Reaktion, harzartige Oxydationsprodukte und säurehaltigen Beimengungen enthält,
wird mit Wasser bis zur neutralen Reaktion gewaschen. Dann

wird die Benzollösung über Natriumsulfat getrocknet, gefiltert und das Benzol wird abgetrieben. Der Rückstand wird bei einer Temperatur von 120 bis 140°C und bei einem Druck von 0,0133 kPa mit dem Ziel der Entfernung des nichtumgesetzten Ausgangsmethylesters abdestilliert. Den Rückstand nach der Destillation kristallisiert man aus Isopropanol beziehungsweise aus n-Hexan aus, wobei man das Endprodukt mit einem Schmelzpunkt ($T_{Schm.}$) gleich 123 bis 125°C erhält (US, A, 3644482).

Bekannt ist ebenfalls ein Verfahren zur Ausscheidung von Pentaerythryl-tetrakis- [3-(3,5-ditert-butyl-4-oxphenil) propionat] aus einer Reaktionsmasse, die bei der Synthese des genannten Produktes durch Umesterung des Methylesters der 3-(3,5-ditert.Butyl-4-oxyphenyl)propionsäure mit Pentaerythrit in Gegenwart eines alkalischen Katalysators, Alkalimetallalkoxids, beispielsweise, Natriummethylats, Lithiummethylats, Lithiumäthylats, gewonnen wird, das darin besteht, daß man der genannten Reaktionsmasse wasserfreie Essigsäure zusetzt, dann man der hergestellten Mischung 95%iges Isopropanol hinzufügt. Aus der hergestellten Lösung wird das Endprodukt bei einer Temperatur von 5°C auskristallisiert, das man durch Filterung abscheidet und mit 95%igem Isopropanol und dann mit Wasser wäscht. Den kristallinen Rückstand des Endproduktes löst man in n-Heptan auf. Die hergestellte Lösung wäscht man einmal mit Wasser und dann entfernt man das Wasser mittels azeotroper Destillation. Aus der getrockneten Heptanlösung wird durch Kristallisation bei einer Temperatur von 5°C das Endprodukt vom Schmelzpunkt $T_{Schm.}$ gleich 121 bis 122°C ausgeschieden (GB, C, 1081789).

Die genannten Verfahren zeichnen sich durch eine Mehrstufigkeit, eine lange Dauer der Prozeßführung, durch die Notwendigkeit, wasserfreie Essigsäure zu verwenden, und durch mehrfache Waschungen mit Wasser, die zum Anfallen einer großen Menge von Abwasser führen, sowie durch die Notwendigkeit, die Lösungen vom Wasser entweder über einem festen Sorptionsmittel oder mittels azeotroper Destillation zu trocknen, was die Prozeßführung verkompliziert und zusätzliche materielle und energetische Aufwendungen verursacht.

Offenbarung der Erfindung

Der Erfindung liegt die Aufgabe zugrunde, im Verfahren zur Ausscheidung von Pentaerythryl-tetrakis- [3-(3,5-ditert.butyl-4-oxyphenyl)propionat] aus einer Reaktionsmasse, die bei der Synthese des genannten Produktes durch Umesterung des Methylesters der 3-(3,5-ditert.Butyl-4-oxyphenyl)propionsäure mit Pentaerythrit in Gegenwart eines alkalischen Katalysators gewonnen wird, ein solches organisches Lösungsmittel und solche Verhältnisse der Prozeßführung zu wählen, die es ermöglichen, die Dauer der Prozeßführung bei einer effektiven Ausscheidung des Endproduktes zu reduzieren, die Verwendung der wasserfreien Essigsäure auszuschließen, die Waschungen mit Wasser und die Trocknung der Lösungen vom Wasser auszuschließen und dadurch die Anzahl der Stufen zu reduzieren, die Entstehung von Abwasser auszuschließen sowie materielle und energetische Aufwendungen herabzusetzen.

Diese Aufgabe wird dadurch gelöst, daß ein Verfahren zur Ausscheidung von Pentaerythryl-tetrakis- [3-(3,5-ditert.butyl-4-oxyphenyl)propionat] aus einer Reaktionsmasse vorgeschlagen wird, die bei der Synthese des genannten Produktes durch Umesterung des Methylesters der 3-(3,5-ditert.Butyl-4-oxyphenyl)propionsäure mit Pentaerythrit in Gegenwart eines alkalischen Katalysators gewonnen wird und die sich aus dem Endprodukt, den nichtumgesetzten Ausgangsreaktionsteilnehmer, den Nebenprodukten der Reaktion, den harzartigen Oxydationsprodukten und den Rückständen des alkalischen Katalysators zusammensetzt, das die Behandlung der Reaktionsmasse mit einem organischen Lösungsmittel und die Ausscheidung des Endproduktes durch Kristallisation aus normalen Kohlenwasserstoffen vorsieht, wobei man erfindungsgemäß als organisches Lösungsmittel Zyklohexan verwendet, die Behandlung der Reak-

- 4 -

tionsmasse mit Zyklohexan bei einem Volumenverhältnis der Reaktionsmasse zum Zyklohexan gleich 1:1,2 bis 1:2 bei einer Temperatur der Reaktionsmasse von 100 bis 120°C durchführt, man nach der genannten Behandlung den nichtumgesetzten Ausgangspentaerythrit durch Filterung und die Rückstände des alkalischen Katalysators bei einer Temperatur von 60 bis 75°C ausscheidet, wobei man eine Zyklohexanlösung aus dem Endprodukt, dem nichtumgesetzten Ausgangsmethylester der 3-(3,5-ditert.Butyl-4-oxyphenyl)propionsäure, den Nebenprodukten der Reaktion und den harzartigen Oxydationsprodukten erhält, dann man die genannte Zyklohexanlösung auf eine Temperatur von 15 bis 20°C unter Anfallen eines kristallinen Rückstandes einer Einschlußverbindung des Endproduktes mit Zyklohexan abkühlt, den genannten kristallinen Rückstand durch Filterung abscheidet, wonach man den kristallinen Rückstand der Einschluß-verbindung des Endproduktes mit Zyklohexan in normalen Kohlenwasserstoffen bei einer Temperatur von 45 bis 75°C und bei einem Massenverhältnis des genannten kristallinen Rückstandes zu den normalen Kohlenwasserstoffen gleich 1:1 bis 1:2 auflöst, wobei sich die genannte Einschlußverbindung unter Anfallen des Endproduktes zersetzt, und die anschließende Ausscheidung des Endproduktes durch Kristallisation aus den normalen Kohlenwasserstoffen bei einer Temperatur von 15 bis 20°C erfolgt.

Durch die Verwendung von Zyklohexan als organisches Lösungsmittel in dem erfindungsgemäßen Verfahren wird eine effektive Abscheidung des nichtumgesetzten Ausgangspentaerythrits und der Rückstände des alkalischen Kataly-sators von der Zyklohexanlösung erreicht. Es wird außer-dem eine effektive Trennung des Endproduktes von den Ne-benbestandteilen (des nichtumgesetzten Ausgangsmethyl-esters, den Nebenprodukten der Reaktion und den harzartigen Oxydationsprodukten) sogar bei einem geringen Gehalt (beispielsweise von 25 Masse%) an Endprodukt in der Reaktionsmasse erreicht. Das erfolgt dadurch, daß sich das Endprodukt aus Zyklohexan in Form einer Einschlußver-

- 5 -

bindung mit Zyklohexan leicht auskristallisieren läßt, während die genannten Nebenbestandteile keine ähnlichen Einschlußverbindungen mit Zyklohexan bilden und in der Lösung bleiben.

Das erfindungsgemäße Verfahren ermöglicht es, die Verwendung der wasserfreien Essigsäure auszuschließen, die Waschungen mit Wasser und die Trocknung der Lösungen vom Wasser auszuschließen und dadurch die Anzahl der Stufen sowie die Dauer der Prozeßführung zu reduzieren, das heißt die Prozeßführung zu vereinfachen und zu intensivieren, sowie die Entstehung von Abwasser auszuschließen und die materiellen und energetischen Aufwendungen herabzusetzen. Das Verfahren ist in technologischer und apparativer Gestaltung einfach und bewirkt die Ausscheidung eines Endproduktes hoher Qualität vom Schmelzpunkt ($T_{Schm.}$) gleich 120 bis 124°C.

Wie oben erwähnt, wird die Behandlung der Reaktionsmasse mit dem Zyklohexan in dem erfindungsgemäßen Verfahren bei einem Volumenverhältnis der Reaktionsmasse zum Zyklohexan gleich 1:1,2 bis 1:2 und einer Temperatur der Reaktionsmasse von 100 bis 120°C vorgenommen.

Die Verwendung von Zyklohexan bei seinem Volumenverhältnis zur Reaktionsmasse unter 1,2:1 wird im Zusammenhang mit der Vergrößerung der Viskosität der Zyklohexanlösung nicht empfohlen, was die Kristallisation der Einschlußverbindung des Endproduktes mit Zyklohexan aus derselben erschwert. Die Verwendung von Zyklohexan bei seinem Volumenverhältnis zur Reaktionsmasse über 2:1 ist infolge eines nichtrationellen Verbrauchs des Zyklohexans und der Verringerung der Prozeßleistung unzweckmäßig.

Die Durchführung der Behandlung der Reaktionsmasse mit Zyklohexan bei einer Temperatur der Reaktionsmasse unter 100°C wird infolge der Erhöhung der Viskosität der Reaktionsmasse nicht empfohlen, was ihre Auflösung im Zyklohexan erschwert. Die Durchführung der Behandlung der Reaktionsmasse mit dem Zyklohexan bei einer Temperatur der Reaktionsmasse über 120°C ist im Zusammenhang mit einer starken Erhöhung des Druckes im System unzweckmäßig.

Nach der genannten Behandlung der Reaktionsmasse mit Zyklohexan scheidet man den nichtumgesetzten Ausgangspentaerythrit und Rückstände des alkalischen Katalysators bei einer Temperatur von 60 bis 75°C durch Filterung ab. Die Durchführung der Filtration bei einer Temperatur unter 60°C ist im Zusammenhang mit der Vergrößerung der Viskosität der Zyklohexanlösung unzweckmäßig, was den Filterungsprozeß erschwert. Die Durchführung der Filtration bei einer Temperatur über 75°C wird im Zusammenhang mit einem starken Druckanstieg im System unzweckmäßig.

Wie oben erwähnt, wird die Zyklohexanlösung, die das Endprodukt, den nichtumgesetzten Ausgangsmethylester, die Nebenprodukte der Reaktion und die harzartigen Oxydationsprodukte enthält, auf eine Temperatur von 15 bis 20°C unter Anfallen eines kristallinen Rückstandes einer Einschlußverbindung des Endproduktes mit Zyklohexan abgekühlt. Die Abkühlung der Lösung auf eine Temperatur unter 15°C wird im Zusammenhang mit einer nichtrationellen Erhöhung des Stromverbrauchs unzweckmäßig. Die Abkühlung der Lösung auf eine Temperatur oberhalb von 20°C ist im Zusammenhang mit der Vergrößerung des Verlustes am Endprodukt unzweckmäßig.

Der angefallene kristalline Rückstand der Einschlußverbindung des Endproduktes mit Zyklohexan löst man in normalen Kohlenwasserstoffen bei einer Temperatur von 45 bis 75°C und bei einem Massenverhältnis des genannten kristallinen Rückstandes zu den normalen Kohlenwasserstoffen gleich 1:1 bis 1:2 auf.

Die Auflösung des genannten kristallinen Rückstandes bei einer Temperatur unter 45°C ist im Zusammenhang mit einem starken Ansteigen der Auflösungsdauer des Rückstandes unzweckmäßig. Die Auflösung des genannten kristallinen Rückstandes bei einer Temperatur von oberhalb 75°C wird im Zusammenhang mit einem starken Ansteigen des Druckes im System nicht empfohlen.

Es wird nicht empfohlen, normale Kohlenwasserstoffe bei ihrem Massenverhältnis zum kristallinen Rückstand der genannten Einschlußverbindung unter 1:1 im Zusammenhang mit der Vergrößerung der Viskosität der Lösung zu verwenden, was die Kristallisation des Endproduktes aus derselben erschwert. Die Verwendung von normalen Kohlenwasserstoffen bei dem Massenverhältnis zum kristallinen Rückstand der genannten Einschlußverbindung über 2:1 ist infolge eines unrationellen Verbrauchs des Lösungsmittels und der Senkung der Prozeßleitung unzweckmäßig.

Wie oben erwähnt, erfolgt die Ausscheidung des Endproduktes durch Kristallisation aus normalen Kohlenwasserstoffen bei einer Temperatur von 15 bis 20°C. Die Durchführung der Kristallisation bei einer Temperatur unter 15°C wird im Zusammenhang mit nichtrationaller Vergrößerung des Stromverbrauchs nicht empfohlen. Die Durchführung der Kristallisation bei einer Temperatur über 20°C ist im Zusammenhang mit der Vergrößerung des Verlustes am Endprodukt unzweckmäßig.

Beste Ausführungsvariante der Erfindung

Das erfindungsgemäße Verfahren zur Ausscheidung von Pentaerythrÿl-tetrakis- [3-(3,5-ditert.butyl-4-oxyphenol) propionat] aus einer Reaktionsmasse, die bei der Synthese des genannten Produktes gewonnen wird, wird wie folgt durchgeführt.

Die Reaktionsmasse, die durch Umesterung des Methylesters der 3-(3,5-ditert.Butyl-4-oxyphenyl)propionsäure mit Pentaerythrit in Gegenwart eines alkalischen Katalysators, beispielsweise, Lithiumhydrids, Natriummethylats, Lithiummethylats, Lithiumäthylats, gewonnen wird und die sich aus dem Endprodukt, den nichtumgesetzten Ausgangsreaktionsteilnehmern, den Nebenprodukten der Reaktion, dem Pentaerythryl-mono- [3-(3,5-ditert.butyl-4-oxyphenyl) propionat] , dem Pentaerythryl-bis- [3-(3,5-ditert. butyl-4-oxyphenyl)propionat] und Pentaerythryl-tris- [3-(3,5-ditert.butyl-4-oxyphenyl)propionat] , sowie aus den harzartigen Oxydationsprodukten und den Rückständen des alkalischen Katalysators zusammensetzt, wird unter Vermischen mit Zyklohexan bei einem Massenverhältnis der Reaktionsmasse zum Zyklohexan gleich 1:1,2 bis 1:2 und einer Temperatur der Reaktionsmasse von 100 bis 120°C behandelt. Dabei sinkt die Temperatur des Gemisches spontan bis 60 bis 75°C. Dann erfolgt die Trennung des nichtumgesetzten Ausgangspentaerythrits und der Rückstände des alkalischen Katalysators von der Zyklohexanlösung an einem Filter bei einer Temperatur von 60 bis 75°C, die das Endprodukt, den nichtumgesetzten Ausgangsmethylester, die Nebenprodukte der Reaktion und die harzartigen Oxydationsprodukte enthält. Die genannte Zyklohexanlösung wird in einen Kristallisator aufgegeben, der mit einem Mischer und einem Thermometer versehen ist, auf eine Temperatur von 15 bis 20°C abgekühlt, und bei der genannten Temperatur vorzugsweise während 6 Stunden stehengelassen, wodurch man einen kristallinen Rückstand einer Einschlußverbindung des Endproduktes mit dem Zyklohexan erhält. Der angefallene kristalline Rückstand wird durch Filterung abgeschieden, an einem Filter mit Zyklohexan gewaschen und in den Kristallisator, der mit einem Mischer und einem Thermometer versehen ist, übertragen, in dem der genannte Rückstand in normalen Kohlenwasserstoffen, beispielsweise, im n-Hexan, n-Heptan, n-Oktan, im Benzin, bei einer Temperatur von 45 bis 75°C und einem Massenverhältnis des genannten kristallinen Rückstandes zu den normalen Kohlenwasserstoffen gleich 1:1 bis 1:2 aufgelöst wird. Unter den genannten Bedingungen zersetzt sich die Einschlußverbindung des Endproduktes mit Zyklohexan unter Anfallen des Endproduktes. Die Lösung des Endproduktes in den normalen Kohlenwasserstoffen wird auf eine Temperatur von 15 bis 20°C abgekühlt und bei den genannten Temperaturwerten vorzugsweise während 5 Stunden stehengelassen, wodurch man kristallinen Rückstand des Endproduktes erhält. Der angefallene kristalline Rückstand wird durch Filterung abgeschieden, an einem Filter mit normalen Kohlenwasserstoffen gewaschen und getrocknet. Hierdurch erhält man das Endprodukt in Form von weißen Kristallen mit einem Schmelzpunkt ($T_{Schm.}$) von 120 bis 124°C.

Zur besseren Erläuterung der vorliegenden Erfindung werden nachstehende Beispiele für ihre konkrete Ausführung angeführt.

Beispiel 1

Man scheidet das Pentaerythryl-tetrakis- [3-(3,5-ditert.butyl-4-oxyphenyl)propionat] aus einer Reaktionsmasse, die bei der Synthese des genannten Produktes durch Umesterung des Methylesters der 3-(3,5-ditert.Butyl-4-oxyphenyl)propionsäure mit Pentaerythrit in Gegenwart eines alkalischen Katalysators, des Lithiumhydrids, gewonnen wird, aus. Die Zusammensetzung der Reaktionsmasse ist wie folgt in Masse%: Pentaerythryl-tetrakis- [3-(3,5-ditert.butyl-4-oxyphenyl)propionat] -- 77,32, nichtumgesetzter Ausgangsmethylester der 3-(3,5-ditert.Butyl-4-oxyphenyl)propionsäure -- 4,0, Pentaerythryl-tris- [3-(3,5-ditert.butyl-4-oxyphenyl)propionat] -- 14,0, Pentaerythryl-bis- [3-(3,5-ditert.butyl-4-oxyphenyl)propionat] -- 0,90, Pentaerythryl-mono- [3-(3,5-ditert.butyl-4-oxyphenyl)propionat] -- 0,10, harzartige Oxydationsprodukte -- 0,77, nichtumgesetzter Ausgangspentaerythrit und Rückstände des alkalischen Katalysators -- 2,91.

Die genannte Reaktionsmasse in einer Menge von 50 cm³ wird unter Vermischen mit 75 cm³ Zyklohexan (Volumenverhältnis der Reaktionsmasse zum Zyklohexan ist gleich 1:1,5) bei einer Temperatur der Reaktionsmasse von 120°C behandelt. Dabei sinkt die Temperatur des Gemisches spontan bis 75°C. Dann wird bei der genannten Temperatur an einem Filter der nichtumgesetzte Ausgangspentaerythrit und Rückstände des alkalischen Katalysators von der Zyklohexanlösung abgeschieden. Dann wird die Zyklohexanlösung in einen Kristallisator, der mit einem Mischer und einem Thermometer versehen ist, aufgegeben, auf eine Temperatur von 20°C abgekühlt und bei der genannten Temperatur für 6 Stunden stehengelassen, wodurch man den kristallinen Rückstand einer Einschlußverbindung des Endproduktes mit Zyklohexan erhält. Der kristalline Rückstand wird durch Filterung abgeschieden und an einem Filter mit 50 cm³ Zyklohexan gewaschen. Nach der Waschung beträgt die Masse des genannten Rückstandes 45,3 g. Der angefallene kristalline Rückstand der Einschlußverbindung des Endproduktes mit dem Zyklohexan wird in einen Kristallisator, der mit einem Mischer und einem Thermometer versehen ist, übertragen, in dem man den genannten Rückstand in 45,3 g Benzin (Massenverhältnis des Rückstandes zum Benzin 1:1) bei einer Temperatur von 75°C auflöst. Unter den genannten Verhältnissen zersetzt sich die Einschlußverbindung des Endproduktes mit Zyklohexan unter Anfallen des Endproduktes. Die Lösung des Endproduktes im Benzin wird auf eine Temperatur von 15°C abgekühlt und bei der genannten Temperatur für 5 Stunden stehengelassen, wodurch man den kristallinen Rückstand des Endproduktes erhält. Der angefallene kristalline Rückstand wird durch Filterung abgeschieden, an einem Filter mit 30 cm$^3$ Benzin gewaschen und getrocknet. Dadurch erhält man 32,85 g Endprodukt in Form von weißen Kristallen vom Schmelzpunkt ($T_{Schm.}$) von 123 bis 124°C.

Beispiel 2

Man scheidet das Pentaerythryl-tetrakis- [3-(3,5-ditert.butyl-4-oxyphenyl)propionat] aus einer Reaktionsmasse ab, die bei der Synthese des genannten Produktes durch Umesterung des Methylesters der 3-(3,5-ditert.Butyl-4-oxyphenyl)propionsäure mit Pentaerythrit in Gegenwart eines alkalischen Katalysators, des Natriummethylats, gewonnen wird. Die Zusammensetzung der Reaktionsmasse ist wie folgt in Masse%: Pentaerythryl-tetrakis- [3-(3,5-ditert.butyl-4-oxyphenyl)propionat] -- 60,23, nichtumgesetzter Ausgangsmethylester der 3-(3,5-ditert.Butyl-4-oxyphenyl)propionsäure -- 0,50, Pentaerythryl-tris- [3-(3,5-ditert.butyl-4-oxyphenyl)propionat] -- 25,40, Petaerythryl-bis- [3-(3,5-ditert.butyl-4-oxyphenyl)propionat] -- 2,70, Pentaerythryl-mono- [3-(3,5-ditert.butyl-4-oxyphenyl)propionat] -- 0,23, harzartige Oxydationsprodukte -- 7,84, nichtumgesetzter Ausgangspentaerythrit und Rückstände des alkalischen Katalysators -- 3,10.

Die genannte Reaktionsmasse in einer Menge von 50 $cm^3$ wird unter Vermischen mit 100 $cm^3$ Zyklohexan (Volumenverhältnis der Reaktionsmasse zum Zyklohexan ist gleich 1:2) bei einer Temperatur der Reaktionsmasse von 100°C behandelt. Dabei verringert sich die Temperatur des Gemisches spontan bis zu 60°C. Dann wird der nichtumgesetzte Ausgangspentaerythrit und Rückstände des alkalischen Katalysators von der Zyklohexanlösung an einem Filter bei der genannten Temperatur abgeschieden. Dann wird die Zyklohexanlösung in einen Kristallisator, der mit einem Mischer und einem Thermometer versehen ist, aufgegeben, auf eine Temperatur von 15°C abgekühlt und bei der genannten Temperatur für 6 Stunden stehengelassen, wodurch man den kristallinen Rückstand einer Einschlußverbindung des Endproduktes mit Zyklohexan erhält. Der kristalline Rückstand wird durch Filterung abgeschieden und an einem Filter mit 50 $cm^3$ Zyklohexan gewaschen. Nach der Waschung beträgt die Masse des genannten Rückstandes 34,8 g.

Der angefallene kristalline Rückstand der Einschlußverbindung des Endproduktes mit dem Zyklohexan wird in einen Kristallisator, der mit einem Mischer und einem Thermometer versehen ist, übertragen, in dem der genannte Rückstand in 41,8 g n-Hexan (Massenverhältnis des Rück-standes zum n-Hexan ist gleich 1:1,2) bei einer Temperatur von 45°C aufgelöst wird. Unter den genannten Bedingungen zersetzt sich die Einschlußverbindung des Endproduktes mit Zyklohexan unter Anfallen des Endproduktes. Die Lösung des Endproduktes im n-Hexan wird auf eine Temperatur von 18°C abgekühlt und bei der genannten Temperatur für 5 Stunden stehengelassen, wodurch man einen kristallinen Rückstand des Endproduktes erhält. Der hergestellte kristalline Rückstand wird durch Filterung abgeschieden, an einem Filter mit 30 $cm^3$ n-Hexan gewaschen und getrocknet. Dadurch erhält man 35,28 g Endprodukt in Form von weißen Kristallen vom Schmelzpunkt ($T_{Schm.}$) von 121 bis 122°C.

Beispiel 3

Man scheidet das Pentaerythryl-tetrakis- [3-(3,5-ditert.butyl-4-oxyphenyl)propionat] aus einer Reaktions-

- 12 -

masse ab, die bei der Synthese des genannten Produktes durch Umesterung des Methylesters der 3-(3,5-ditert.Butyl--4-oxyphenyl)propionsäure mit Pentaerythrit in Gegenwart eines alkalischen Katalysators, Lithiumäthylats, gewonnen wird. Die Zusammensetzung der Reaktionsmasse ist wie folgt in Masse%: Pentaerythryl-tetrakis- [3-(3,5-ditert.butyl--4-oxyphenyl)propionat] -- 40,03, nichtumgesetzter Ausgangsmethylester der 3-(3,5-ditert.Butyl-4-oxyphenyl) propionsäure -- 7,50, Pentaerythryl-tris- [3-(3,5-ditert. butyl-4-oxyphenyl)propionat] -- 36,86, Pentaerythryl-bis- [3-(3,5-ditert.butyl-4-oxyphenyl)propionat] -- 7,32, Pentaerythryl-mono- [3-(3,5-ditert.butyl-4-oxy-phenyl)propionat] -- 1,32, harzartige Oxydationsprodukte -- 3,67, nichtumgesetzter Ausgangspentaerythrit und Rückstände des alkalischen Katalysators -- 2,8.

Die genannte Reaktionsmasse in einer Menge 50 $cm^3$ wird unter Vermischen mit 60 $cm^3$ Zyklohexan (Volumenver-hältnis der Reaktionsmasse zum Zyklohexan ist gleich 1:1,2) bei einer Temperatur der Reaktionsmasse von 110°C behandelt. Dabei verringert sich die Temperatur des Gemisches spontan bis zu 68°C. Dann wird der nichtumgesetzte Ausgangspentaerythrit und Rückstände des alkalischen Katalysators an einem Filter bei der genannten Temperatur von der Zyklohexanlösung abgeschieden. Dann wird die Zyklohexanlösung in einen Kristallisator, der mit einem Mischer und einem Thermometer versehen ist, aufgegeben, auf eine Temperatur von 15°C abgekühlt und bei der genannten Temperatur für 6 Stunden stehengelassen, wodurch man einen kristallinen Rückstand einer Einschlußverbindung des Endproduktes mit Zyklohexan erhält. Der kristalline Rückstand wird durch Filterung abgeschieden und an einem Filter mit 30 $cm^3$ Zyklohexan gewaschen. Nach der Waschung beträgt die Masse des genannten Rückstandes 22,7 g.

Der angefallene kristalline Rückstand der Einschlußverbindung des Endproduktes mit Zyklohexan wird in den Kristallisator, der mit dem Mischer und dem Thermometer versehen ist, übertragen, in dem der genannte Rückstand in 45,4 g n-Oktan (Massenverhältnis des Rückstandes zum n-Oktan ist gleich 1:2) bei einer Temperatur von 70°C aufgelöst wird. Unter den genannten Bedingungen zersetzt sich die Einschlußverbindung des Endproduktes mit Zyklohexan unter Anfallen des Endproduktes. Die Lösung des Endproduktes in n-Oktan wird auf eine Temperatur von 15°C abgekühlt und bei der genannten Temperatur für 5 Stunden stehengelassen, wodurch man kristallinen Rückstand des Endproduktes erhält. Der angefallene kristalline Rückstand wird durch Filterung abgeschieden, an einem Filter mit 20 cm$^3$ n-Oktan gewaschen und getrocknet. Dadurch erhält man 16,4 g Endprodukt in Form von weißen Kristallen vom Schmelzpunkt (T$_{Schm.}$) gleich 120 bis 121°C.

Beispiel 4

Man scheidet das Pentaerythryl-tetrakis- [3-(3,5-ditert.butyl-4-oxyphenyl)propionat] aus einer Reaktionsmasse ab, die bei der Synthese des genannten Produktes durch Umesterung des Methylesters der 3-(3,5-ditert. Butyl-4-oxyphenyl)propionsäure mit Pentaerythrit in Gegenwart eines alkalischen Katalysators, Natriumhydroxids, gewonnen wird. Die Zusammensetzung der Reaktionsmasse ist wie folgt in Masse%: Pentaerythryl-tetrakis- [3-(3,5-ditert.butyl-4-oxyphenyl)propionat] -- 25,0, nichtumgesetzter Ausgangsmethylester der 3-(3,5-ditert.butyl-4-oxyphenyl)propionsäure -- 14,2, Pentaerythryl-tris- [3-(3,5-ditert.butyl-4-oxyphenyl)propionat] -- 35,5, Pentaerythryl-bis- [3-(3,5-ditert.butyl-4-oxyphenyl)propionat] -- 10,8, Pentaerythryl-mono- [3-(3,5-ditert.butyl-4-oxyphenyl)propionat] -- 4,0, harzartige Oxidationsprodukte -- 7,0, nichtumgesetzter Ausgangspentaerythrit und Rückstände des alkalischen Katalysators -- 3,5.

Die genannte Reaktionsmasse in einer Menge von 50 cm$^3$ wird unter Vermischen mit 60 cm$^3$ Zyklohexan (Volumenverhältnis der Reaktionsmasse zum Zyklohexan ist gleich 1:1,2) bei einer Temperatur der Reaktionsmasse von 120°C behandelt. Dabei verringert sich die Temperatur

- 14 -

des Gemisches spontan bis zu 75°C. Dann wird der nichtumgesetzte Ausgangspentaerythrit und Rückstände des alkalischen Katalysators an einem Filter bei der genannten Temperatur von der Zyklohexanlösung abgeschieden. Dann wird die Zyklohexanlösung in einen Kristallisator, der mit einem Mischer und einem Thermometer versehen ist, aufgegeben, auf eine Temperatur von 18°C abgekühlt und bei der genannten Temperatur für 6 Stunden stehengelassen, wodurch man den kristallinen Rückstand einer Einschlußverbindung des Endproduktes mit Zyklohexan erhält. Der kristalline Rückstand wird durch Filterung abgeschieden und an einem Filter mit 20 cm$^3$ Zyklohexan gewaschen. Nach der Waschung beträgt die Masse des genannten Rückstandes 12,9 g. Der angefallene kristalline Rückstand der Einschlußverbindung des Endproduktes mit Zyklohexan wird in den Kristallisator, der mit einem Mischer und einem Thermometer versehen ist, übertragen, in dem der genannte Rückstand in 12,9 g Benzin (Massenverhältnis des Rückstandes zum Benzin ist gleich 1:1) bei einer Temperatur von 75°C aufgelöst wird. Unter den genannten Bedingungen zersetzt sich die Einschlußverbindung des Endproduktes mit Zyklohexan unter Anfallen des Endproduktes. Die Lösung des Endproduktes in Benzin wird auf einer Temperatur von 20°C abgekühlt und bei der genannten Temperatur für 5 Stunden stehengelassen, wodurch man den kristallinen Rückstand des Endproduktes erhält. Der angefallene kristalline Rückstand wird durch Filterung abgeschieden, an einem Filter mit 10 cm$^3$ Benzin gewaschen und getrocknet. Dadurch erhält man 9,38 g Endprodukt in Form von weißen Kristallen vom Schmelzpunkt ($T_{Schm.}$) gleich 121 bis 122°C.

Hierdurch ermöglicht es das erfindungsgemäße Verfahren, bei einer effektiven Ausscheidung des Endproduktes aus Reaktionsmassen mit einem unterschiedlichen Gehalt an Endprodukt in denselben die Anzahl der Stufen und die Dauer des Ausscheidungsprozesses zu reduzieren, die Entstehung von Abwasser auszuschließen sowie materielle und energetische Aufwendungen herabzusetzen.

Industrielle Anwendbarkeit

Die vorliegende Erfindung kann auf dem Gebiet der organischen Synthese zur Ausscheidung von Pentaerythryl-tetrakis- [3-(3,5-ditert.butyl-4-oxyphenyl)propionat] aus einer Reaktionsmasse, die bei der Synthese des genannten Produktes durch Umesterung des Methylesters der 3-(3,5-ditert.Butyl-4-oxyphenyl)propionsäure mit Pentaerythrit in Gegenwart eines alkalischen Katalysators gewonnen wird, ihre Anwendung finden.

Das genannte Endprodukt ist ein hocheffektives nichttoxisches Antioxydationsmittel der Polyolefine, des Polystyrols und anderer Polymerstoffen, die die genannten Polymere nicht färben.

- 16 -

PATENTANSPRUCH

Verfahren zur Ausscheidung von Pentaerythryl-tetrakis- [3-(3,5-ditert.butyl-4-oxyphenyl)propionat] aus einer Reaktionsmasse, die bei der Synthese des genannten Produktes durch Umesterung des Methylesters der 3-(3,5-ditert. Butyl-4-oxyphenyl)propionsäure mit Pentaerythrit in Gegenwart eines alkalischen Katalysators gewonnen wird und die sich aus dem Endprodukt, den nichtumgesetzten Ausgangsreaktionsteilnehmern, den Nebenprodukten der Reaktion, den harzartigen Oxydationsprodukten und den Rückständen des alkalischen Katalysators zusammensetzt, das die Behandlung der Reaktionsmasse mit einem organischen Lösungsmittel und die Ausscheidung des Endproduktes durch Kristallisation aus normalen Kohlenwasserstoffen vorsieht, d a d u r c h  g e k e n n z e i c h n e t, daß man als organisches Lösungsmittel Zyklohexan verwendet, die Behandlung der Reaktionsmasse mit Zyklohexan bei einem Volumenverhältnis der Reaktionsmasse zum Zyklohexan gleich 1:1,2 bis 1:2 und einer Temperatur der Reaktionsmasse von 100 bis 120°C durchführt, nach der genannten Behandlung den nichtumgesetzten Ausgangspentaerythrit und Rückstände des alkalischen Katalysators durch Filterung bei einer Temperatur von 60 bis 75°C abgeschieden wird, wobei man die Zyklohexanlösung aus dem Endprodukt, dem nichtumgesetzten Ausgangsmethylester der 3-(3,5-ditert. Butyl-4-oxyphenyl)propionsäure, den Nebenprodukten der Reaktion und den harzartigen Oxydationsprodukten erhält, dann die genannte Zyklohexanlösung auf eine Temperatur von 15 bis 20°C unter Anfallen eines kristallinen Rückstandes einer Einschlußverbindung des Endproduktes mit Zyklohexan abgekühlt, der genannte kristalline Rückstand durch Filterung abgeschieden wird, wonach man den kristallinen Rückstand der Einschlußverbindung des Endproduktes mit Zyklohexan in normalen Kohlenwasserstoffen bei einer Temperatur von 45 bis 75°C und bei einem Massenverhältnis des genannten kristallinen Rückstandes zu den normalen Kohlenwasserstoffen gleich 1:1 bis 1:2 auflöst, wobei sich die genannte Einschlußverbindung unter Anfallen des Endproduktes zersetzt, und die anschließende
Ausscheidung des Endproduktes durch Kristallisation aus
normalen Kohlenwasserstoffen bei einer Temperatur von 15
bis 20°C erfolgt.

# INTERNATIONAL SEARCH REPORT

International Application No PCT/SU 87/00144

## I. CLASSIFICATION OF SUBJECT MATTER (if several classification symbols apply, indicate all)

According to International Patent Classification (IPC) or to both National Classification and IPC

IPC$^4$ — C 07 C 69/732, 67/52

## II. FIELDS SEARCHED

### Minimum Documentation Searched [7]

| Classification System | Classification Symbols |
|---|---|
| IPC$^4$ | C 07 C 67/48, 67/52, 69/732, 69/75 |

Documentation Searched other than Minimum Documentation
to the Extent that such Documents are Included in the Fields Searched [8]

## III. DOCUMENTS CONSIDERED TO BE RELEVANT [9]

| Category [*] | Citation of Document, [11] with indication, where appropriate, of the relevant passages [12] | Relevant to Claim No. [13] |
|---|---|---|
| A | EP, B1, 0066189, (MITSUI PETROCHEMICAL INDUSTRIES, LTD.), 1 August 1984 (01.08.84) | 1 |
| A | JP, B1, 42-18617, 23 September 1967 (23.09.67) | 1 |
| A | GB, A, 1103144, (J.R. GEIGY A.-G.) 14 February 1968 (14.02.68) | 1 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 15 August 1988 (15.08.88) | 1 September 1988 (01.09.88) |

| International Searching Authority | Signature of Authorized Officer |
|---|---|
| ISA/SU | |